# EUROPEAN PATENT SPECIFICATION

(11) **EP 2 680 791 B1**
(45) Date of publication and mention of the grant of the patent: **04.05.2016**
(21) Application number: 11860065.9
(22) Date of filing: 03.03.2011
(51) Int. Cl.: A61F 2/24, A61F 2/958, A61F 2/966, A61M 25/10

(54) **TEMPORARY PERFUSION CHANNEL PERCUTANEOUS DELIVERY OF BALLOON-EXPANDABLE STENTS**
PERKUTANE FREISETZUNG EINES BALLONEXPANDIERBAREN STENTS MIT EINEM TEMPORÄREN PERFUSIONSKANAL
CANAL DE PERFUSION TEMPORAIRE POUR ADMINISTRATION PERCUTANÉE D'ENDOPROTHÈSES VASCULAIRES DILATABLES PAR BALLONNET

(43) Date of publication of application: 08.01.2014
(73) Proprietor: Empire Technology Development LLC, Wilmington, DE 19808 (US)
(72) Inventor: LESCHINSKY, Boris, Mahwah NJ 07430 (US)
(74) Representative: Dennemeyer & Associates S.A.
(86) International application number: PCT/US2011/027023
(87) International publication number: WO 2012/118508

(56) References cited:
- US-A- 5 755 708
- US-A1- 2003 100 939
- US-A1- 2007 073 391
- US-A1- 2008 114 439
- US-A1- 2008 221 552
- US-A1- 2009 228 093
- US-A1- 2009 264 988

## Description

### BACKGROUND

Unless otherwise indicated herein, the materials described in this section are not prior art to the claims in this application and are not admitted to be prior art by inclusion in this section.

Some of the common diseases of the cardio-vascular system include atherosclerosis, diseased or damaged heart valves, aortic or thoracic aneurysms, and similar ones. One of the therapeutic approaches for atherosclerosis or aortic / thoracic aneurysms is placement of a permanent stent or stent graft at the diseased location. A stent graft is a tubular device, which is composed of special fabric supported by a rigid structure, usually metal. The rigid structure is called a stent. A typical stent may be formed from a metal mesh. Stent grafts may also be used in endovascular surgery to support weak points in arteries or veins, commonly known as an aneurysm. Once in place inside the aorta, the stent graft may act as a false lumen for blood to travel through, instead of flowing into the aneurysm sack.

Another use for stents is aortic valve replacement. The aortic valve can be affected by a range of diseases and the valve can either become leaky or partially blocked. Aortic valve may be replaced through open heart surgery. Instead of open heart surgery, a catheter-based approach (percutaneous aortic valve replacement or PAVR), may be employed to replace a damaged heart valve with a valved stent.

The present disclosure appreciates that there are several limitations with percutaneous delivery systems for stents and/or stent grafts. In non-limiting example, a common deployment mechanism for stents is a balloon placed inside the stent that is inflated to deploy the stent (or stent graft) and then deflated for removal from the blood vessel. When the balloon is inflated during deployment, however, blood flow through the artery or vein may be substantially reduced. Similarly, in aortic valve replacement procedures, the blood flow may be interrupted from the left ventricle resulting in strong hemodynamic forces being applied on the delivery system. The hemodynamic forces, in return, may cause the stent or replacement valve to be dislodged from its optimal position. The blood flow interruption and resulting hemodynamic forces may also necessitate rapid deployment of the stent or replacement valve to avoid compromising blood circulation.

Document US 5 755 708 A discloses a mechanical prosthesis deployment device for enlarging a flow passage of a vessel by delivering and deploying an expandable stent or prosthesis within an obstruction in the vessel. The prosthesis deployment device comprises a cylindrically shaped expansion member adapted to be disposed within the expandable prosthesis and includes a means engaged to the expansion member for altering the distance between the proximal end and the distal end of the expansion member thereby transforming the expansion member between a diametrically contracted configuration and a diametrically expanded configuration.

### SUMMARY

This summary is provided to introduce a selection of concepts in a simplified form that are further described below in the Detailed Description. This summary is not intended to exclusively identify key features or essential features of the claimed subject matter, nor is it intended as an aid in determining the scope of the claimed subject matter.

The present disclosure describes a continuous circulation percutaneous stent delivery system according to claim 1. Further advantageous features are defined in the dependent claims.

### BRIEF DESCRIPTION OF THE DRAWINGS

The following and other features of this disclosure will become more fully apparent from the following description and appended claims, taken in conjunction with the accompanying drawings. Understanding that these drawings depict only several embodiments in accordance with the disclosure and are, therefore, not to be considered limiting of its scope, the disclosure will be described with additional specificity and detail through use of the accompanying drawings, in which:
FIG. 1 is a conceptual diagram illustrating a percutaneous delivery system with temporary perfusion channel use;
FIGS. 2A, 2B, and 2C illustrate an example delivery of a stent through an expandable balloon;
FIG. 3 illustrates possible effects of hemodynamic forces on a percutaneous delivery system for balloon-expandable replacement valves;
FIG. 4 illustrates an example continuous circulation percutaneous delivery system with the central perfusion channel collapsed;
FIG. 5 illustrates the example continuous circulation percutaneous delivery system of

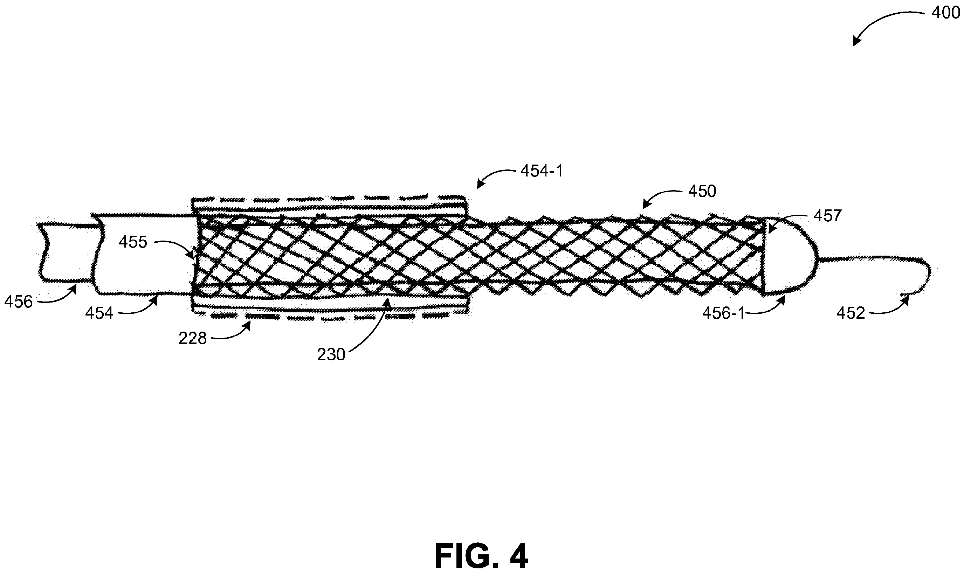

FIG. 4 with the central perfusion channel expanded;

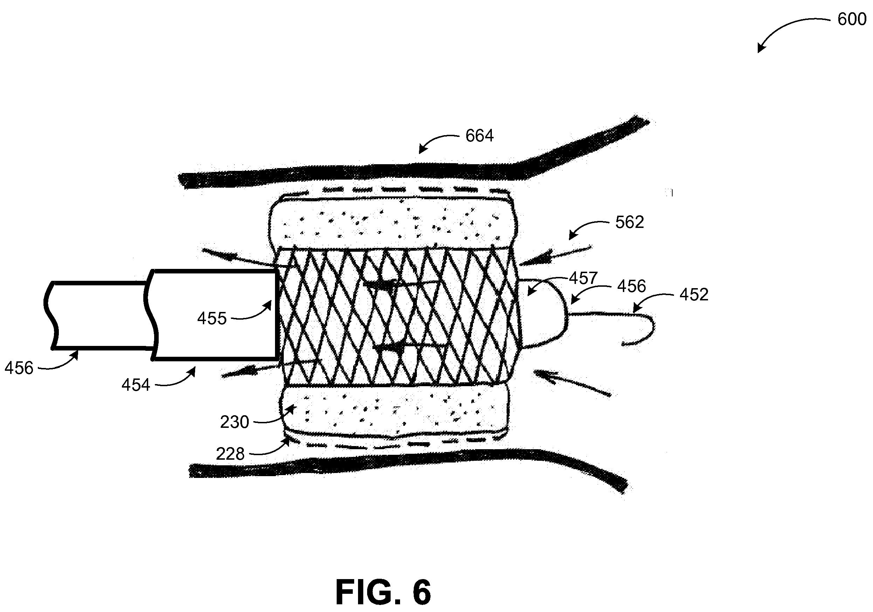

FIG. 6 illustrates the example continuous circulation percutaneous delivery system of

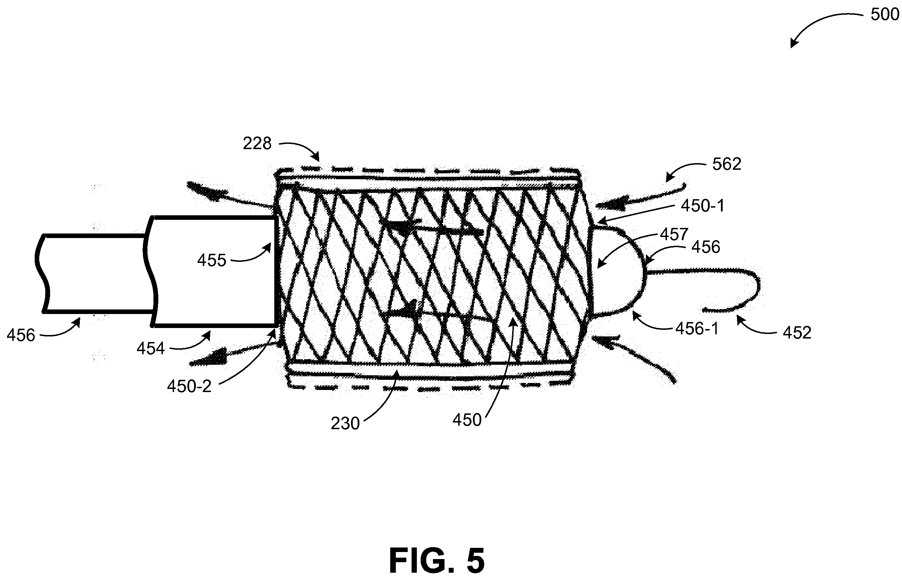

FIG. 5 with the stent deployed through inflation of the balloon;

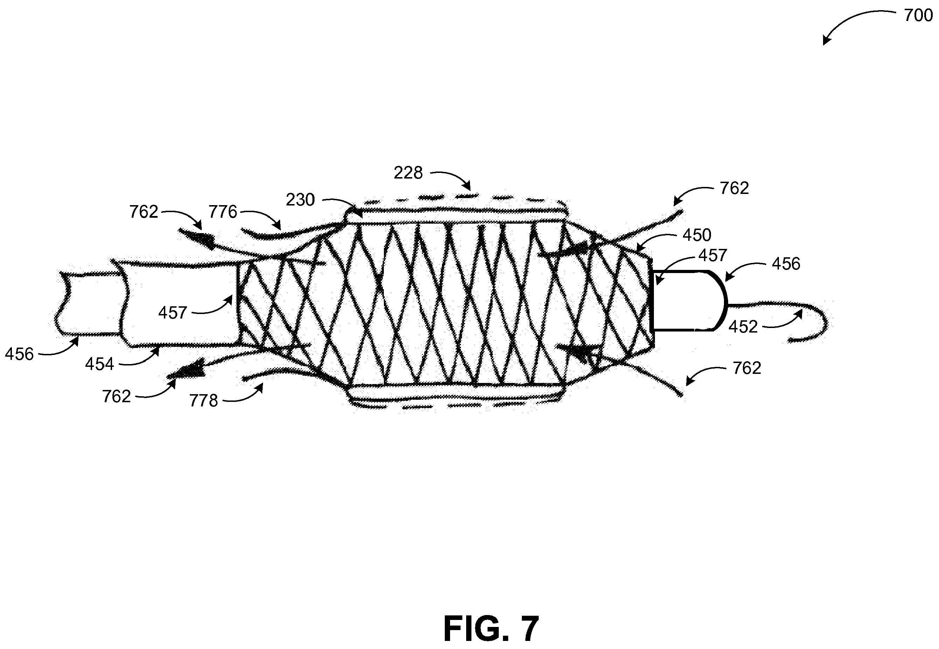

FIG. 7 illustrates the example continuous circulation percutaneous delivery system of
FIG. 5 with temporary valve leaflets for unidirectional flow control; and

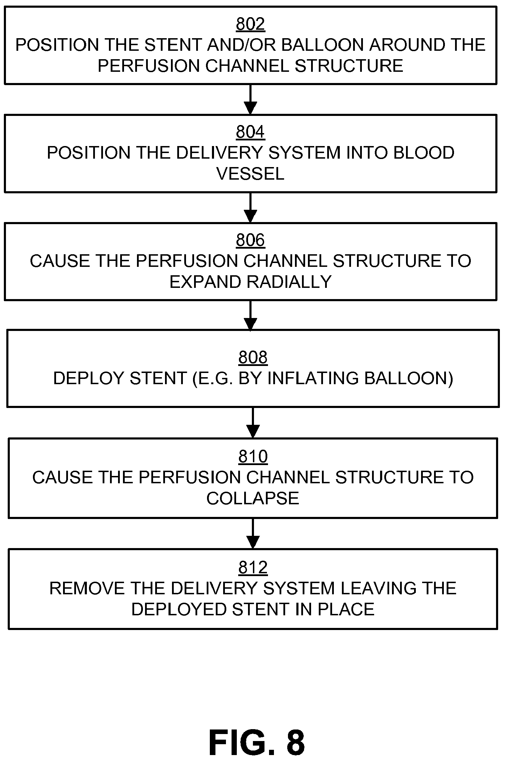

FIG. 8 illustrates a flowchart for a process of deploying a stent employing a continuous circulation percutaneous delivery system with a central perfusion channel,
all arranged in accordance with at least some embodiments described herein.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein.

This disclosure is generally drawn, inter alia, to methods, apparatus, systems, devices, and/or computer program products related to percutaneous delivery systems for stents and replacement valves with continuous blood flow.

Briefly stated, technologies are generally described for percutaneous valve delivery systems with continuous blood flow. By providing a temporary perfusion channel in balloon-expandable stent and/or valve replacement systems, blood flow during the deployment of the stent or valve, when the inflation of the balloon may interrupt the blood flow, is ensured. The perfusion channel may be formed employing an elongated braided structure, which may be expanded radially and outwards upon being longitudinally compressed. One-way blood flow through the channel may be accomplished by providing a temporary check valve.

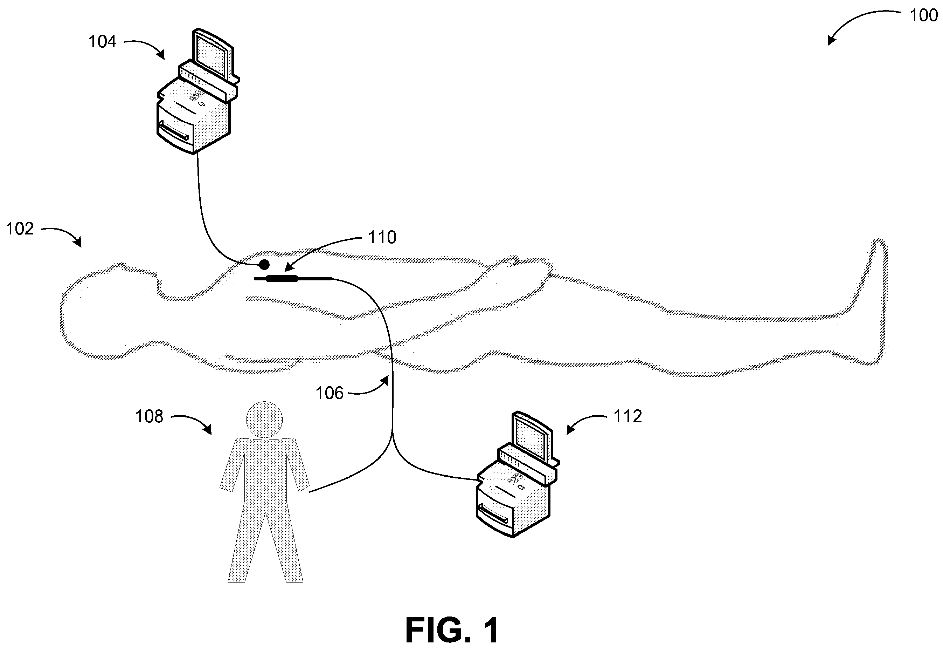

FIG. 1 is a conceptual diagram illustrating a percutaneous delivery system with temporary perfusion channel use in accordance with some embodiments described herein.

Aortic or mitral valve damage, atherosclerosis, and aortic or thoracic aneurysms are some of the common diseases of the cardio-vascular system. Conventional surgical therapies involving replacement of valves or shunting of diseased blood vessel portions are increasingly being replaced with percutaneous delivery of artificial valves or stent grafts. Surgical approaches are typically associated with procedures like opening of the pericardium, use of cardio-pulmonary bypass machine, post-surgical stay in intensive care units, and prolonged recovery periods. Thus, risks to patients can be significant at various stages of the therapy.

In contrast, percutaneous delivery procedures may be performed without full anesthesia and in some case as an outpatient procedure. Post-surgical recovery with percutaneous delivery procedures may also be substantially faster compared to recovery following surgical approaches, which may last as long as a year. Diagram 100 illustrates, conceptually, a percutaneous delivery procedure, where a physician 108 may insert a percutaneous delivery system 106 (typically a catheter or similar mechanism) carrying the stent or replacement valve through an artery or vein, and guide it to a proper location (diseased blood vessel or damaged heart valve) by monitoring the progress of the delivery system through a monitoring device 104. Monitoring device 104 may enable the physician 108 to observe the progress of the delivery system in the patient's body 102 employing X-ray, ultrasound, or similar monitoring mechanisms.

The percutaneous delivery system may carry a balloon-inflatable replacement valve stent or a regular stent (110) for expanding a clogged blood vessel or supporting a blood vessel weakened by an aneurysm. The delivery system may include one or more probes for monitoring blood pressure, blood oxygen level, and other chemical properties of the blood through monitoring device 112. In a system according to some embodiments, the delivery system may be equipped with a temporary perfusion channel to enable continuous blood flow while the balloon is being inflated to deploy the stent or replacement valve.

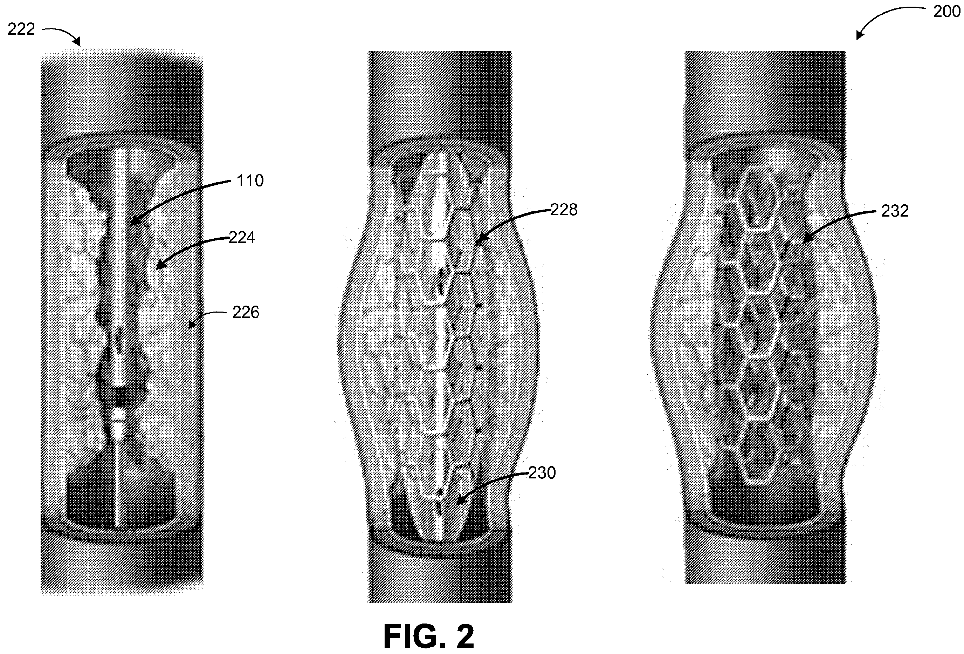

FIG. 2A, 2B, and 2C illustrate an example delivery of a stent through an expandable balloon 200 in accordance with some conventional methods.

As shown in FIG. 2A, a stent delivery mechanism may include a catheter (or guide wire) 110 with the stent and a deployment mechanism (e.g., a balloon). The catheter 110 may be guided through the blood vessel 222 to the proper location such as, but not limited to, an at least partially occluded section 224 of the blood vessel, where an atherosclerotic plaque may be present inside the arterial (or venous) wall 226. Once in place, (FIG. 2B) the stent 228 may be deployed, for example, by inflating the balloon 230 inside the stent structure. The deployed stent (FIG. 2C) may push the arterial wall 226 radially outward forming a channel 232 for increased blood flow. Upon deployment of the stent, the balloon may be deflated and removed from the blood vessel by pulling out the catheter 110.

Cardio-vascular stents may be made from flexible, supportive, and biocompatible materials capable of expansion. Mesh structures are commonly used. Foreign materials implanted into the human body may result in trauma, inflammation, immune response and eventual healing and/or scarring. Materials that are not biocompatible can induce many complications, including cytotoxic chemical buildup and chronic inflammation. Stents are commonly built on a stainless steel platform due to cost and manufacturability considerations. However, stainless steel is not fully compatible with the human body and implantation may be followed by restenosis and thrombosis. In addition, stainless steel can pose difficulties related to some types of imaging, such as magnetic resonance. Alternative platform materials such as but not limited to gold, titanium, cobalt-chromium alloy, tantalum alloy, nitinol (55% nickel and 45% titanium), and different types of polymers may also be used as stent materials.

While certain polymers have found use as stent materials such as but not limited to silicone, which induces low rates of tissue trauma, they also present challenges in terms of biodurability, tensile and coil strength, and inner-to-outer diameter. Biodegradable or bioabsorbable stents may include a component (such as an enzyme or microbe) that degrades the stent material quickly enough to make them appropriate for short-term uses. A bioerodible polymer is a water-insoluble polymer that has been converted into a water-soluble material. Biodegradable materials can form an effective stent coating because they can be mixed with an antirestenotic drug and can degrade within a few weeks, thus releasing the drug into the surrounding tissue and reducing the risk of restenosis. Examples of biodegradable polymers include, but are not limited to: polyesters, polyorthoesters and polyanhydrides. Shape-memory polymers can be used to produce a device that can transition from a temporary state to a different (permanent) state through the inducement of a stimulus of heat or cold, for example.

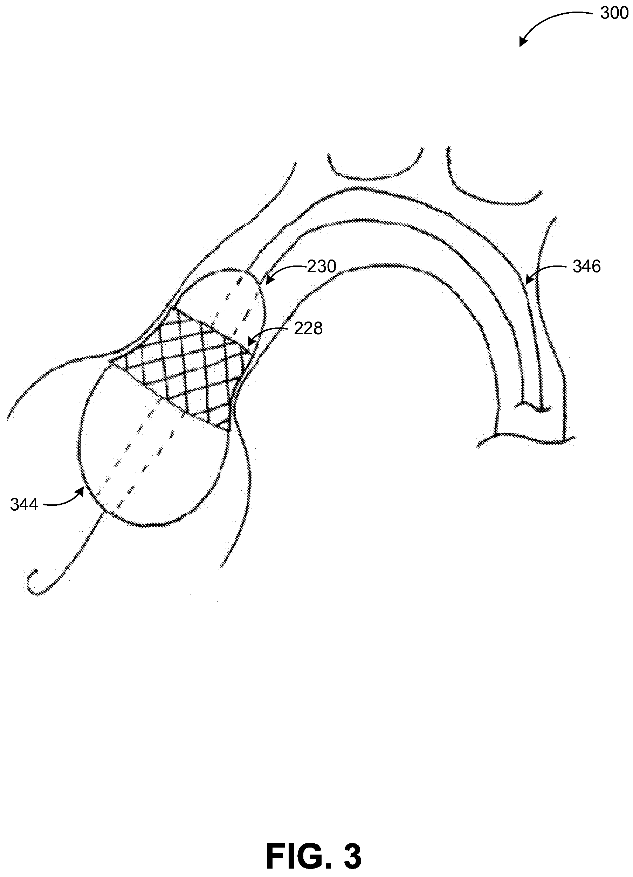

FIG. 3 illustrates possible effects of hemodynamic forces on a conventional percutaneous delivery system for balloon-expandable replacement valves.

In percutaneous valve replacement (aortic or mitral) a synthetic or tissue (human or animal) valve is inserted to the heart typically through the femoral artery from a small entry wound in the groin. The diseased valve, which may have become leaky (e.g., aortic insufficiency/regurgitation) or partially blocked (e.g., aortic stenosis) for example, may then be replaced with the healthy one. The procedure is comparable to that performed when placing a stent, or performing balloon angioplasty. Percutaneous valve replacement removes the need for open heart surgery, which is an invasive procedure with considerable mortality and morbidity especially in more fragile patients.

As illustrated in diagram 300, a valved stent 228 may be placed strategically near the diseased valve by the delivery mechanism 346 (e.g., a catheter-based system). A balloon 230 inside the valved stent 228 may be inflated, deploying the stent. As shown in FIG. 3, one side of the balloon (344) may typically inflate more than the other side, almost completely blocking blood flow during deployment. This interruption of blood ejection from the left ventricle may result in strong hemodynamic forces on the deployed stent, which may cause suboptimal valve positioning. Furthermore, the deployment may have to be completed rapidly to prevent damage such as myocardial infarction or stroke to the patient.

In a system according to at least some embodiments, accurate placement of a percutaneous aortic valve with no or minimal pressure gradient from the ventricle to the aorta portion distal of the valve may be accomplished through a temporary perfusion channel. Minimal pressure may be defined in example circumstances as less than about 10 mm Hg pressure. In illustrative embodiments, assuming a 25 mm diameter aorta, the force on the valve may not exceed about 0.23 kg (0.5 pound). The channel may be expanded at the center of the balloon prior to the valve being deployed enabling continuous blood flow prior to, during, and after the deployment. By selecting dimensions (diameters) of the temporary perfusion channel structure and the stent deployment system (balloon and stent itself) accordingly, the interruption in blood flow may be minimized. The balloon may be selected as thin and as small as possible when in compressed state. In illustrative embodiments, the perfusion channel may have a diameter between about 5 mm and about 20 mm in expanded state.

FIG. 4 illustrates an example continuous circulation percutaneous delivery system 400 with the central perfusion channel collapsed.

An expandable perfusion channel 450 is provided prior to deploying a valved (or regular) stent 228. The perfusion channel 450 may be expanded prior to valve deployment to occupy from about 20 percent to about 80 percent of the cross-section of the aortic annulus. A temporary check valve is provided to assure the flow through the perfusion channel to be unidirectional. For example, in case of aortic valve, the flow may be from the left ventricle to the aorta. The valved stent 228 may be located (or placed in a separate step) around the expanded perfusion channel. The stent 228 may then be deployed either by a self-expansion mechanism or by a balloon 230 expansion mechanism. The balloon 230 in this case may be designed with a central opening matching the dimension of the perfusion channel 450 when expanded. Inflation of the balloon 230 is designed to cause expansion of the valved stent 228, which creates a radially inward pressure on the perfusion channel 450. Thus, the perfusion channel 450 may be designed to withstand the radial pressure created by the balloon. For example, a depth of the material forming the perfusion channel 450, size and/or shape of braids in a braided perfusion channel, or comparable properties may be selected based on expected radial pressure. In illustrative implementations, the perfusion channel may be selected to withstand expected balloon pressures between about 68.95 kPa (10 PSI) and 137.9 kPa (20 PSI).

In contrast with conventional stent deployment procedures, where due to the hemodynamic forces the deployment needs to be completed within a brief period of time (e.g., 5 minutes or less), use of the perfusion channel described herein allows stent deployment to progress at a slower pace (e.g., 30 minutes), if needed, as blood flow from the ventricle is largely uninterrupted. The relatively longer length of available time may allow for frequent verification of positioning using fluoroscopy or other appropriate imaging. In addition, strong hemodynamic forces may be avoided during deployment decreasing a risk of displacement of the valved stent 228.

The delivery system for the valved stent may include a central catheter 456 which may be inserted into the left ventricle over a guide wire 452. An outer catheter 454 may be placed over the central catheter 456 such that it can slide over the central catheter controlled by a person performing the deployment procedure. In diagram 400, a distal end 456-1 (in relation to a person deploying the stent) of the central catheter 456 is shown having an approximately round terminus, which is typically preferred to avoid damage to tissue as the catheter is inserted. However, central catheter 456 and outer catheter 454 may have any shape of termini, varying sizes, and be made of any appropriate material known in the art. As shown in diagram 400, an example configuration includes outer catheter 454 over central catheter 456, perfusion channel 450 over outer catheter 454 with one end of the perfusion channel attached to a distal end 456-1 of the central catheter 456 at attachment location 457 and the other end of the perfusion channel attached to the outer catheter 454 at attachment location 455. Deployment balloon 230 is positioned over the perfusion channel 450 and the valved stent 228 is positioned over the deployment balloon 230, all in a collapsed state. The deployment balloon 230 and the valved stent 228 may be positioned anywhere over the perfusion channel 450 prior to insertion into the body or slid over the perfusion channel after it is in place in a blood vessel. Proximal ends of the central and outer catheters 456 and 454 (not shown) are typically outside the body.

Perfusion channel 450 is formed by a braided structure which expands radially when the structure is compressed longitudinally and collapses radially when the structure is expanded longitudinally. A proximal end of the perfusion channel (braided structure) may be attached (e.g. through adhesion or mechanical attachment) to a suitable location on the outer catheter 454. For example, the perfusion channel may be crimped over, retained by a sleeve, encapsulated within overmolding, or attached using other appropriate methods. The suitable location for attachment may take a length of the deployment balloon 230 and valved stent 228 into account. As shown by the example attachment location 455 in diagram 400, the deployment balloon 230 and valved stent 228 in their collapsed state may be placed over the collapsed perfusion channel 450. In other embodiments, the perfusion channel may extend proximally (and optionally distally) beyond the length of the balloon and/or stent over the outer catheter 454. In yet other embodiments, the perfusion channel 450 may be slid over the outer catheter after the catheters are in place within the blood vessel and deployed through the use of the guide wire or similar mechanism (e.g. not attached to a fixed location on the central or outer catheters).

An example of a braided structure that can be elongated to pass through a small space (e.g., blood vessel) is a mesh-based stent. Such structures function to reinforce an outer structure and prevent a collapse. In such structures, a radial strength of the structure is usually a function of an angle of the braid. When the angle approaches 90 degrees to the axis of the braid the radial strength approaches its maximum. Retaining the stent at both ends further increases the resistance of the braid to withstand a radial force applied towards the stent's center. The elongated braided structure forming the perfusion channel 450 may be composed of one or more of gold, titanium, a cobalt-chromium alloy, a tantalum alloy, stainless steel, nitinol, silicon, a biodegradable polymer, a bioabsorbable polymer, and/or a bioerodible polymer. An outer diameter of the structure may be selected to be less than a diameter of the blood vessel to allow expansion room for the stent and/or balloon deploying the stent. The elongated braided structure may also include a coating on the outside to allow it to adhere to the balloon temporarily as the stent or valve is being deployed. The braids may be formed by a metallic or other material in triangular, rectangular, or polygonal shapes enabling the structure to expand and collapse longitudinally while collapsing and expanding radially at the same time.

In a deployment process, the example elongated braided structure may be compressed longitudinally by sliding the central catheter 456 (or outer catheter 454) such that the distal ends 456-1 and 454-1 approach each other. The compression may cause a radial expansion due to the braided nature of the structure (individual shapes compress in one direction expand in another as a result). Thus, the elongated structure may expand to its operative dimensions within the blood vessel by longitudinal compression. The perfusion channel 450 may preserve its diameter substantially during the deployment of the stent or valve (e.g., inflation of the balloon) by maintenance of the longitudinal compression (i.e., fixed position of the catheters relative to each other). Upon completion of the deployment, the longitudinal compression may be removed or an opposite direction force applied such that the elongated structure collapses to its compressed diameter for removal from the blood vessel. In other examples, shape memory properties of materials used to form the braided structure may promote the return to a preselected shape after being released from the longitudinally compressed position. Some examples of shape memory materials include, but are not limited to, copper-zinc-aluminum-nickel, copper-aluminum-nickel, nickel-titanium (NiTi), zinc-copper-gold alloys, and block copolymers such as polyethylene terephthalate (PET) - polyethyleneoxide (PEO), polystyrene - poly(1,4-butadiene), poly(2-methyl-2-oxazoline) - polytetrahydrofuran.

While reference is made to valved stent, a perfusion channel as described herein may be used in deployment of any stent in circulatory system. A valved stent is just one example. The balloon and stent may be located centrally or toward one end of the perfusion channel in collapsed state. Additionally, the balloon and the stent may also be placed behind or in front of the perfusion channel 450 and configured to allow their placement over the perfusion channel 450 once at the desired deployment location.

FIG. 5 illustrates the example continuous circulation percutaneous delivery system of FIG. 4 with the central perfusion channel expanded.

As discussed above, a delivery system for a valved stent 228 may include a central catheter 456 over a guide wire 452 and an outer catheter 454 configured to slide over the central catheter 456. The perfusion channel 450 may be formed as a braided structure placed over the central catheter 456 with a distal end 450-1 of the braided structure attached to the distal end 456-1 of the central catheter 456 and a proximal end 450-2 of the braided structure attached to the outer catheter 454.

As shown in diagram 500, slidingly moving at least one of the catheters such that their distal ends approach each other causes longitudinal compression of the braided structure, which in turn may cause the perfusion channel 450 to expand radially outwards. Expansion of the braided structure creates an opening for blood flow 562 through a center of the perfusion channel 450. An open wire expanded braided structure may allow blood flow to go from the right side to the left side (e.g., from the left ventricle to the aorta in aortic valve replacement procedures). According to some embodiments, a length of the expanded braided structure may be selected to at least match or exceed a length of the deployment balloon 230 such that full support is provided for the balloon during its inflation. In illustrative embodiments, a length of the perfusion channel when expanded may range between about 12.7 mm (½ inch) and 38.1 mm (1½ inches). The length when collapsed may range from about 25.4 mm (1 inch) to about 127 mm (5 inches). The diameter of the perfusion channel in expanded state may range between about 5 mm and about 30 mm. Mesh size may vary from about ½ mm to about 7 mm.

FIG. 6 illustrates the example continuous circulation percutaneous delivery system of FIG. 5 with the stent deployed through inflation of the balloon.

Diagram 600 depicts the stent delivery system discussed above with the stent deployment balloon 230 inflated and the valved stent 228 expanded towards the walls 664 of the aortic annulus. The perfusion channel 450 is held in expanded position by maintaining the relative locations of central and outer catheters 456 and 454, which through the attachment locations 457 and 455, respectively, determine a length of the perfusion channel, and thereby a diameter as discussed above. Continuous circulation 562 is provided through the deployment location by the expanded perfusion channel 450. Once the valved stent 228 is deployed, the balloon 230 may be deflated and the perfusion channel 450 is collapsed by slidingly moving the central and outer catheters (456, 454), which elongates the braided structure of the perfusion channel 450 through respective attachment locations 457 and 455 back to its low profile state. The system may then be withdrawn from the patient. FIG. 7 illustrates the example continuous circulation percutaneous delivery system of FIG. 5 with temporary valve leaflets for unidirectional flow control according to some embodiments described herein.

As shown in diagram 700, unidirectional flow through the opening of the central perfusion channel 450 may be provided by incorporating a set of collapsible temporary check valves 776 and 778 therewith. The collapsible temporary check valves 776, 778 may be made from the same or similar material as the perfusion channel 450 (e.g., metal or polymer, but without the mesh structure). The collapsible temporary check valves 776, 778 may be formed in various shapes such as round, elongated, rectangular, elliptical, etc. and may be attached to the perfusion channel 450 through flexible adhesion or similar manner that enables the check valves to move in order to allow blood flow through the openings which the valves cover. A size of the collapsible temporary check valves 776, 778 may be selected depending on a size of the perfusion channel 450. For example, the valves may cover an area that ranges from about 1/4 of the diameter of the perfusion channel to about 2/3 of the diameter of the perfusion channel. According to some embodiments, the braided structure of the perfusion channel may be formed with a gradual slope on one or both of its ends by having a smaller expanded diameter value for the structures end(s) compared to its longitudinal center (e.g., the diameters may have a ratio ranging from about 1 to 1.5 to about 1 to 3). The slope(s) may provide for a convenient check valve supporting structure. The flexible leaflets of a temporary valve 776, 778 may be placed at one end (left end as shown in the diagram) and configured to collapse about the sloping end of the braided structure to prevent back flow. During ejection from the left ventricle these leaflets may open up the passages for blood flow 762. During the heart diastole, the leaflets may fall back around the braided structure and at least partially or completely prevent blood regurgitation back into the left ventricle. Other locations for a check valve may include the other end (right on diagram 700) or any location inside the braided structure.

A system as shown in diagram 700 may be implemented for balloon-expandable valves, which are typically delivered by expanding a valved stent at the location of a native aortic valve. The stent itself may contain the leaflets of a replacement valve. In addition, embodiments may be implemented in percutaneous delivery systems of other devices expandable in the arterial or venous vessels of the circulatory system. In particular, percutaneous devices according to embodiments may be employed for deployment of stent/grafts for treatment of thoracic and abdominal aortic aneurysms.

While embodiments have been discussed above using specific examples, components, and configurations, they are intended to provide a general guideline to be used for delivering stents, stent grafts, and/or replacement valves within the cardio-vascular system with uninterrupted blood circulation. These examples do not constitute a limitation on the embodiments, which may be implemented using other components, delivery system configurations, delivery schemes, and/or materials using the principles described herein.

FIG. 8 illustrates a flowchart for a process of deploying a stent employing a continuous circulation percutaneous delivery system with a central perfusion channel arranged in accordance with at least some embodiments described herein.

A process of deploying a stent employing a continuous circulation percutaneous delivery system with a central perfusion channel may optionally begin with operation 802, "POSITION THE STENT AND/OR BALLOON AROUND THE PERFUSION CHANNEL STRUCTURE." At operation 802, a regular or valved stent 228 may be positioned around a perfusion channel 450 along with a deployment mechanism such as an inflatable balloon 230. The stent and the balloon may be placed near one end of the perfusion channel 450, around the middle of the perfusion channel 450, or moved over the perfusion channel 450 once the delivery system is at its delivery location.

Operation 802 may be followed by operation 804, "POSITION THE DELIVERY SYSTEM INTO BLOOD VESSEL." At operation 804, the delivery system 110 may be inserted into a major blood vessel and guided through the patient's body to the delivery location such as the location of a damaged valve or diseased blood vessel (e.g., aneurysm). In some embodiments, the perfusion channel 450 may be positioned in the blood vessel first and the stent and/or balloon deployed around the perfusion channel 450 (in collapsed status).

Operation 804 may be followed by operation 806, "CAUSE THE PERFUSION CHANNEL STRUCTURE TO EXPAND RADIALLY." At operation 806, the perfusion channel 450 may be expanded radially, for example, by longitudinally compressing a braided structure forming the channel. The expanded perfusion channel 450 may enable continuous blood flow prior to, during, and after the deployment of the stent 228.

Operation 806 may be followed by operation 808, "DEPLOY STENT (E.G. BY INFLATING BALLOON." At operation 808, the stent 228 may be deployed through its deployment mechanism. While inflation of a balloon 230 may be used to deploy the stent 228, other mechanisms may also be employed. The perfusion channel 450 may be designed with sufficient radial structural strength to withstand compression forces that may be encountered during the inflation of the balloon 230. Upon deployment of the stent, the balloon may be deflated leaving the stent 228 in place.

Operation 808 may be followed by operation 810, "CAUSE THE PERFUSION CHANNEL STRUCTURE TO COLLAPSE." At operation 810, the perfusion channel 450 may be collapsed by expanding it longitudinally (for example, as illustrated in FIG. 4 and 5 in reverse order). By using a braided structure or similar design, continuous blood flow 562 may be achieved during the expansion and collapse of the perfusion channel 450.

Operation 810 may be followed by operation 812, "REMOVE THE DELIVERY SYSTEM LEAVING THE DEPLOYED STENT IN PLACE." At operation 812, the delivery system 110 may be removed from the patient body through the same blood vessel leaving the deployed stent in place.

The operations discussed above are for illustration purposes. Temporary perfusion channel for percutaneous delivery of balloon-expandable stents may be implemented by similar processes with fewer or additional operations. In some examples, the operations may be performed in a different order. In some other examples, various operations may be eliminated. In still other examples, various operations may be divided into additional operations, or combined together into fewer operations.

A method for providing percutaneous delivery of a stent with continuous circulation may be provided. According to some examples, the method includes positioning a perfusion channel structure in a blood vessel, causing the perfusion channel structure to expand such that blood flow is enabled through the perfusion channel structure, positioning a stent over the perfusion channel structure, and deploying the stent.

According to other examples, the method may include causing the perfusion channel structure to collapse and removing the perfusion channel structure from the blood vessel. The stent may be deployed by expanding a balloon placed around the perfusion channel structure and inside the stent. The stent and/or the balloon may be positioned about concentrically over the perfusion channel structure.

According to further examples, the method may also include causing the perfusion structure to expand radially by reducing a length of an elongated braided structure that forms the perfusion channel structure longitudinally, employing a first catheter coupled to one end of the elongated braided structure and extended longitudinally relative to a second catheter, sliding the first catheter longitudinally along the second catheter to expand the elongated braided structure, and re-extending the catheter to collapse the elongated braided structure upon deployment of the stent. The stent may be delivered with a valve system for mitral valve replacement or aortic valve replacement. One-way blood flow may be ensured before, during, and/or after the deployment of the stent by employing a temporary check valve internal to the perfusion channel structure.

In accordance with other embodiments, a percutaneous stent delivery system with continuous circulation may be provided. According to some examples, the percutaneous stent delivery system may include an expandable perfusion channel structure, a first guide configured to deliver the perfusion channel structure into a blood vessel and a second guide configured to expand the perfusion channel structure by reducing a length of the perfusion channel structure prior to, during, and/or following deployment of a stent such that blood flow is enabled through the perfusion channel structure as the stent is deployed. The percutaneous stent delivery system may also include the stent configured to be positioned over the perfusion channel structure.

According to other examples, the stent may be deployable through one of a self-expansion mechanism or a balloon expansion mechanism. The stent and the balloon expansion mechanism may be positioned around at least one of a proximal end, a distal end, or a middle of the perfusion channel structure. The balloon expansion mechanism may include a central opening approximately matching a radial dimension of the expanded perfusion channel structure. Moreover, the perfusion channel structure may be configured to withstand a radial pressure during the expansion of the balloon without collapsing. At least one of the stent and/or the balloon may be positioned about concentrically over the perfusion channel structure.

According to further examples, the first guide may be a guide wire or a catheter and the second guide may be a guide wire, a catheter, or a pull wire. A length of the expanded perfusion channel structure may be selected to at least match or exceed a length of the balloon expansion mechanism such that full support is provided for the balloon during deployment of the stent. The elongated braided structure may have a substantially tubular elongated shell, with a tubular channel formed within the braided structure. An outer diameter of the structure may be selected to be less than a diameter of the blood vessel to allow expansion room for the stent and/or balloon deploying the stent. The braids may be formed by a metallic or other material in triangular, rectangular, or polygonal shapes enabling the structure to expand and collapse longitudinally while collapsing and expanding radially at the same time. The perfusion channel structure may include an elongated braided structure that is composed of one or more of stainless steel, gold, titanium, a cobalt-chromium alloy, a tantalum alloy, nitinol, silicon, a biodegradable polymer, a bioabsorbable polymer, and/or a bioerodible polymer.

The elongated structure may be compressed to a diameter that is smaller than its expanded diameter, and may be placed in the working channel of a catheter for delivery to the desired location. When the elongated structure is deployed from the catheter, it may expand to its operative dimensions within the blood vessel by longitudinal compression. The braided structure may preserve its diameter substantially during the deployment of the stent or valve (e.g., inflation of the balloon) by maintenance of the longitudinal compression. Upon completion of the deployment, the longitudinal compression may be removed or an opposite direction force applied such that the elongated structure collapses to its compressed diameter for removal from the blood vessel. In other examples, shape memory properties of materials used to form the braided structure may promote the return to a preselected shape after being released from the longitudinally compressed position.

According to yet other examples, a distal end of the elongated braided structure may be attached to a distal end of the first guide and a proximal end of the elongated braided structure may be attached to a distal end of the second guide such that the elongated braided structure is longitudinally expanded or compressed in response to a sliding motion of the first guide and the second guide. The first and second guides may be concentrically positioned catheters. The expanded perfusion channel structure may occupy between approximately 20% and approximately 80% of a cross-section of the blood vessel annulus. The perfusion channel structure includes a temporary check valve for ensuring one-way blood flow during the deployment of the stent. The temporary check valve may ensure one-way blood flow from a distal end to a proximal end of the perfusion channel structure in an aortic valve replacement or mitral valve replacement. The temporary check valve may include a set of leaflets attached to at least one of a distal end and/or a proximal end of the perfusion channel structure. Furthermore, the stent may include one or more leaflets of a valve for replacing one of an aortic valve or a mitral valve.

In accordance with further embodiments, a percutaneous delivery system with continuous circulation may be provided. The percutaneous delivery system may include a perfusion channel structure configured to be expanded radially prior to deployment of a stent such that blood flow is enabled through the perfusion channel structure prior to, during, and/or following the deployment of the stent; and two concentric guides an outer one of which is coupled to a distal end of the perfusion channel structure such that moving a distal end of the outer guide closer to a distal end of the central guide causes a reduction of a length of the perfusion channel structure and thereby the radial expansion of the perfusion channel structure outwards.

According to some examples, a length of the expanded perfusion channel structure may be selected to at least match or exceed a length of an inflated balloon that is employed to deploy the stent. The perfusion channel structure may include an elongated braided structure composed of one or more of stainless steel, gold, titanium, a cobalt-chromium alloy, a tantalum alloy, nitinol, silicon, a biodegradable polymer, a bioabsorbable polymer, and/or a bioerodible polymer. The perfusion channel structure may include a set of leaflets attached to at least one of a distal end and/or a proximal end of the perfusion channel structure for ensuring one-way blood flow during the deployment of the stent.

According to other examples, the perfusion channel structure may be configured to deploy the stent in at least one of an arterial and/or a venous vessel of the circulatory system. The perfusion channel structure may also be configured to deploy a valved stent to replace one of an aortic valve or a mitral valve. Moreover, the perfusion channel structure may be configured to deploy a stent-graft for treatment of a thoracic or an abdominal aneurysm.

The present disclosure is not to be limited in terms of the particular embodiments described in this application, which are intended as illustrations of various aspects. Many modifications and variations can be made without departing from its scope, as will be apparent to those skilled in the art. Functionally equivalent methods and apparatuses within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds compositions or biological systems, which can, of course, vary. It is also to be understood that the terminology used herein is for the purpose of describing particular embodiments only, and is not intended to be limiting.

Those skilled in the art will recognize that it is common within the art to describe devices and/or processes in the fashion set forth herein, and thereafter use engineering practices to integrate such described devices and/or processes into data processing systems. That is, at least a portion of the devices and/or processes described herein may be integrated into a medical treatment system via a reasonable amount of experimentation. Those having skill in the art will recognize that a typical medical treatment system may generally include one or more additional components such as a system unit housing, a video display device, a memory such as volatile and non-volatile memory, processors such as microprocessors and digital signal processors, injection mechanisms, monitoring mechanisms, and similar components.

The herein described subject matter sometimes illustrates different components contained within, or connected with, different other components. It is to be understood that such depicted architectures are merely illustrative, and that in fact many other architectures may be implemented which achieve the same functionality. In a conceptual sense, any arrangement of components to achieve the same functionality is effectively "associated" such that the desired functionality is achieved. Hence, any two components herein combined to achieve a particular functionality may be seen as "associated with" each other such that the desired functionality is achieved, irrespective of architectures or intermediate components. Likewise, any two components so associated may also be viewed as being ""operably connected", or "operably coupled", to each other to achieve the desired functionality, and any two components capable of being so associated may also be viewed as being "operably couplable", to each other to achieve the desired functionality. Specific examples of operably couplable include but are not limited to physically connectable and/or physically interacting components and/or wirelessly interactable and/or wirelessly interacting components and/or logically interacting and/or logically interactable components.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations).

Furthermore, in those instances where a convention analogous to "at least one of A, B, and C, etc." is used, in general such a construction is intended in the sense one having skill in the art would understand the convention (*e*.*g*., " a system having at least one of A, B, and C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims.

## Claims

1. A continuous circulation percutaneous stent delivery system (700) comprising:
an expandable perfusion channel structure (450) formed by an elongated braided structure and configured to maintain an expanded conformation during stent (228) deployment;
a first guide (452, 454, 456) configured to deliver the perfusion channel structure (450) into a blood vessel;
a second guide (452, 454, 456) configured to expand the perfusion channel structure (450) by reducing a length of the perfusion channel structure (450) and
causing the elongated braided structure to expand radially such that blood flow (762) is enabled through the perfusion channel structure (450); and
a stent (228) configured to be positioned over the expanded perfusion channel structure (450) during deployment,
wherein the perfusion channel structure (450) includes a temporary check valve (776, 778) for ensuring one-way blood flow (762) during the deployment of the stent (228).

2. The continuous circulation percutaneous stent delivery system (700) according to claim 1, wherein the stent (228) is deployable through one of a self-expansion mechanism or a balloon (230) expansion mechanism.

3. The continuous circulation percutaneous stent delivery system (700) according to claim 2, wherein the stent (228) and the balloon(230) expansion mechanism are positioned around at least one of a proximal end, a distal end, or a middle of the perfusion channel structure (450).

4. The continuous circulation percutaneous stent system (700) according to claim 2,wherein the balloon (230) expansion mechanism includes a central opening approximately matching a radial dimension of the expanded perfusion channel structure (450).

5. The continuous circulation percutaneous stent delivery system (700) according to claim 2, wherein the perfusion channel structure (450) is configured to withstand a radial pressure during the expansion of the balloon (230) without collapsing, or wherein at least one of the stent (228) and the balloon (230) are positioned about concentrically over the perfusion channel structure (450).

6. The continuous circulation percutaneous stent delivery system (700) according to claim 1, wherein the first guide is one of a guide wire (452) or a catheter (454, 456) and the second guide is one of a guide wire (452), a catheter (454, 456), or a pull wire.

7. The continuous circulation percutaneous stent delivery system (700) according to claim 2, wherein a length of the expanded perfusion channel structure (450) is selected to at least match or exceed a length of the balloon (230) expansion mechanism such that full support is provided for the balloon (230) during deployment of the stent (228).

8. The continuous circulation percutaneous stent delivery system (700) according to claim 1, wherein the elongated braided structure is composed of one or more of stainless steel, gold, titanium, a cobalt-chromium alloy, a tantalum alloy, nitinol, silicon, a biodegradable polymer, a bioabsorbable polymer, and/or a bioerodible polymer.

9. The continuous circulation percutaneous stent delivery system (700) according to claim 1, wherein a distal end of the elongated braided structure is attached to a distal end of the first guide (452, 454, 456) and a proximal end of the elongated braided structure is attached to a distal end of the second guide (452, 454, 456) such that the elongated braided structure is longitudinally expanded or compressed in response to a sliding motion of the first guide and the second guide.

10. The continuous circulation percutaneous stent delivery system (700) according to claim 1, wherein the first and second guides are concentrically positioned catheters (454, 456).

11. The continuous circulation percutaneous stent delivery system (700) according to claim 1, wherein the expanded perfusion channel structure (450) is configured to occupy between approximately 20% and approximately 80% of a cross-section of the blood vessel annulus.

12. The continuous circulation percutaneous stent delivery system (700) according to claim 1, wherein the temporary check valve (776, 778) is configured to ensure one-way blood flow (762) from a distal end to a proximal end of the perfusion channel structure (450) in one of an aortic valve replacement or mitral valve replacement.

13. The continuous circulation percutaneous stent delivery system (700) according to claim 1, wherein the temporary check valve (776, 778) comprises a set of leaflets attached to at least one of a distal end and/or a proximal end of the perfusion channel structure (450).

14. The continuous circulation percutaneous stent delivery system (700) according to claim 1, wherein the stent (228) includes one or more leaflets of a valve for replacing one of an aortic valve or a mitral valve.

## Patentansprüche

1. Perkutanes Stentapplikationssystem (700) für kontinuierliche Zirkulation, umfassend:
eine expandierbare Perfusionskanalstruktur (450), die durch eine längliche geflochtene Struktur gebildet wird und so ausgelegt ist, dass sie während der Entfaltung des Stents (228) eine expandierte Gestalt beibehält;
eine erste Führung (452, 454, 456), die so ausgelegt ist, dass sie die Perfusionskanalstruktur (450) in ein Blutgefäß einführt;
eine zweite Führung (452, 454, 456), die so ausgelegt ist, dass sie die Perfusionskanalstruktur (450) durch Reduzieren einer Länge der Perfusionskanalstruktur (450) expandiert und bewirkt, dass die längliche geflochtene Struktur radial derart expandiert, dass Blutfluss (762) durch die Perfusionskanalstruktur (450) ermöglicht wird; und
einen Stent (228), der so ausgelegt ist, dass er während der Entfaltung über der expandierten Perfusionskanalstruktur (450) positioniert wird,
wobei die Perfusionskanalstruktur (450) eine temporäre Rückschlagklappe (776, 778) zum Gewährleisten von Blutfluss (762) in einer Richtung während der Entfaltung des Stents (228) umfasst.

2. Perkutanes Stentapplikationssystem (700) für kontinuierliche Zirkulation nach Anspruch 1, wobei der Stent (228) durch einen Selbstexpansionsmechanismus oder einen Ballon (230)-Expansionsmechanismus entfaltet werden kann.

3. Perkutanes Stentapplikationssystem (700) für kontinuierliche Zirkulation nach Anspruch 2, wobei der Stent (228) und der Ballon (230)-Expansionsmechanismus um mindestens eines von einem proximalen Ende, einem distalen Ende oder einer Mitte der Perfusionskanalstruktur (450) positioniert sind.

4. Perkutanes Stentapplikationssystem (700) für kontinuierliche Zirkulation nach Anspruch 2, wobei der Ballon (230)-Expansionsmechanismus eine mittige Öffnung umfasst, die ungefähr einer radialen Abmessung der expandierten Perfusionskanalstruktur (450) entspricht.

5. Perkutanes Stentapplikationssystem (700) für kontinuierliche Zirkulation nach Anspruch 2, wobei die Perfusionskanalstruktur (450) so ausgelegt ist, dass sie einem radialen Druck während der Expansion des Ballons (230) widersteht, ohne zusammenzufallen, oder wobei mindestens einer von dem Stent (228) und dem Ballon (230) etwa konzentrisch über der Perfusionskanalstruktur (450) positioniert ist.

6. Perkutanes Stentapplikationssystem (700) für kontinuierliche Zirkulation nach Anspruch 1, wobei die erste Führung eines von einem Führungsdraht (452) oder einem Katheter (454, 456) ist, und die zweite Führung eines von einem Führungsdraht (452), einem Katheter (454, 456) oder einem Zugdraht ist.

7. Perkutanes Stentapplikationssystem (700) für kontinuierliche Zirkulation nach Anspruch 2, wobei eine Länge der expandierten Perfusionskanalstruktur (450) so ausgewählt ist, dass sie mindestens einer Länge des Ballon (230)-Expansionsmechanismus entspricht oder diese überschreitet, derart dass volle Unterstützung für den Ballon (230) während der Entfaltung des Stents (228) bereitgestellt wird.

8. Perkutanes Stentapplikationssystem (700) für kontinuierliche Zirkulation nach Anspruch 1, wobei die längliche geflochtene Struktur aus einem oder mehreren von Edelstahl, Gold, Titan, einer Cobalt-Chrom-Legierung, einer Tantal-Legierung, Nitinol, Silizium, einem biologisch abbaubaren Polymer, einem biologisch absorbierbaren Polymer und/oder einem biologisch erodierbaren Polymer besteht.

9. Perkutanes Stentapplikationssystem (700) für kontinuierliche Zirkulation nach Anspruch 1, wobei ein distales Ende der länglichen geflochtenen Struktur an einem distalen Ende der ersten Führung (452, 454, 456) befestigt ist, und ein proximales Ende der länglichen geflochtenen Struktur an einem distalen Ende der zweiten Führung (452, 454, 456) befestigt ist, derart dass die längliche geflochtene Struktur in Reaktion auf eine Schiebebewegung der ersten Führung und der zweiten Führung der Länge nach expandiert oder komprimiert wird.

10. Perkutanes Stentapplikationssystem (700) für kontinuierliche Zirkulation nach Anspruch 1, wobei die ersten und zweiten Führungen konzentrisch positionierte Katheter (454, 456) sind.

11. Perkutanes Stentapplikationssystem (700) für kontinuierliche Zirkulation nach Anspruch 1, wobei die expandierte Perfusionskanalstruktur (450) so ausgelegt ist, dass sie zwischen ungefähr 20 % und ungefähr 80 % eines Querschnitts des Blutgefäß-Kreisrings einnimmt.

12. Perkutanes Stentapplikationssystem (700) für kontinuierliche Zirkulation nach Anspruch 1, wobei die temporäre Rückschlagklappe (776, 778) so ausgelegt ist, dass sie Blutfluss (762) in einer Richtung von einem distalen Ende zu einem proximalen Ende der Perfusionskanalstruktur (450) in einem von einem Aortenklappenersatz oder einem Mitralklappenersatz gewährleistet.

13. Perkutanes Stentapplikationssystem (700) für kontinuierliche Zirkulation nach Anspruch 1, wobei die temporäre Rückschlagklappe (776, 778) einen Satz von Klappensegeln umfasst, die an mindestens einem von einem distalen Ende und/oder einem proximalen Ende der Perfusionskanalstruktur (450) befestigt sind.

14. Perkutanes Stentapplikationssystem (700) für kontinuierliche Zirkulation nach Anspruch 1, wobei der Stent (228) ein oder mehrere Klappensegel einer Klappe zum Ersetzen einer von einer Aortenklappe oder einer Mitralklappe umfasst.

## Revendications

1. Système de mise en place d'endoprothèse par voie percutanée à circulation continue (700) comprenant :
une structure de canal de perfusion extensible (450) formée par une structure tressée allongée et configurée pour maintenir une conformation étirée lors du déploiement de l'endoprothèse (228) ;
un premier guide (452, 454, 456) configuré pour mettre en place la structure de canal de perfusion dans un vaisseau sanguin ;
un second guide (452, 454, 456) configuré pour étirer la structure de canal de perfusion (450) en réduisant une longueur de la structure de canal de perfusion (450) et en amenant la structure tressée allongée à s'étirer radialement de telle sorte que l'écoulement sanguin (762) soit possible à travers la structure de canal de perfusion (450) ; et
une endoprothèse (228) configurée pour être positionnée sur la structure de canal de perfusion étirée (450) lors du déploiement,
dans lequel la structure de canal de perfusion (450) comprend un clapet anti-retour temporaire (776, 778) pour assurer un écoulement sanguin unidirectionnel (762) lors du déploiement de l'endoprothèse (228).

2. Système de mise en place d'endoprothèse par voie percutanée à circulation continue (700) selon la revendication 1, dans lequel l'endoprothèse (228) peut être déployée par le biais d'un mécanisme d'auto-extension ou d'un mécanisme d'extension à ballonnet (230).

3. Système de mise en place d'endoprothèse par voie percutanée à circulation continue (700) selon la revendication 2, dans lequel l'endoprothèse (228) et le mécanisme d'extension à ballonnet (230) sont positionnés autour d'au moins l'un d'une extrémité proximale, d'une extrémité distale ou d'un centre de la structure de canal de perfusion (450).

4. Système de mise en place d'endoprothèse par voie percutanée à circulation continue (700) selon la revendication 2, dans lequel le mécanisme d'extension à ballonnet (230) comprend une ouverture centrale correspondant approximativement à une dimension radiale de la structure de canal de perfusion étirée (450).

5. Système de mise en place d'endoprothèse par voie percutanée à circulation continue (700) selon la revendication 2, dans lequel la structure de canal de perfusion (450) est configurée pour résister à une pression radiale lors de la dilatation du ballonnet (230) sans s'écraser, ou dans lequel au moins l'un de l'endoprothèse (228) et du ballonnet (230) sont positionnés de manière sensiblement concentrique sur la structure de canal de perfusion (450).

6. Système de mise en place d'endoprothèse par voie percutanée à circulation continue (700) selon la revendication 1, dans lequel le premier guide est l'un d' un fil-guide (452) ou d'un cathéter (454, 456) et le second guide est l'un d'un fil-guide (452), d'un cathéter (454, 456) ou d'un fil de traction.

7. Système de mise en place d'endoprothèse par voie percutanée à circulation continue (700) selon la revendication 2, dans lequel une longueur de la structure de canal de perfusion étirée (450) est choisie pour au moins correspondre à ou dépasser une longueur du mécanisme d'extension à ballonnet (230) de telle sorte qu'un support total est apporté au ballonnet (230) lors du déploiement de l'endoprothèse (228).

8. Système de mise en place d'endoprothèse par voie percutanée à circulation continue (700) selon la revendication 1, dans lequel la structure tressée étirée est composée d'un ou plusieurs parmi l'acier inoxydable, l'or, le titane, un alliage cobalt-chrome, un alliage de tantale, le nitinol, le silicium, un polymère biodégradable, un polymère bioabsorbable et/ou un polymère bioérodable.

9. Système de mise en place d'endoprothèse par voie percutanée à circulation continue (700) selon la revendication 1, dans lequel une extrémité distale de la structure tressée allongée est fixée à une extrémité distale du premier guide (452, 454, 456) et une extrémité proximale de la structure tressée allongée est fixée à une extrémité distale du second guide (452, 454, 456) de telle sorte que la structure tressée allongée est étirée ou comprimée longitudinalement en réponse à un mouvement coulissant du premier guide et du second guide.

10. Système de mise en place d'endoprothèse par voie percutanée à circulation continue (700) selon la revendication 1, dans lequel les premier et second guides sont des cathéters (454, 456) positionnés de manière concentrique.

11. Système de mise en place d'endoprothèse par voie percutanée à circulation continue (700) selon la revendication 1, dans lequel la structure de canal de perfusion étirée (450) est configurée pour occuper entre environ 20 % et environ 80 % d'une section transversale de l'anneau du vaisseau sanguin.

12. Système de mise en place d'endoprothèse par voie percutanée à circulation continue (700) selon la revendication 1, dans lequel le clapet anti-retour temporaire (776, 778) est configuré pour assurer un écoulement sanguin unidirectionnel (762) d'une extrémité distale à une extrémité proximale de la structure de canal de perfusion (450) dans l'un d'un remplacement de valve aortique ou d'un remplacement de valve mitrale.

13. Système de mise en place d'endoprothèse par voie percutanée à circulation continue (700) selon la revendication 1, dans lequel le clapet anti-retour temporaire (776, 778) comprend un ensemble de feuillets fixés à au moins l'une d'une extrémité distale et/ou d'une extrémité proximale de la structure de canal de perfusion (450).

14. Système de mise en place d'endoprothèse par voie percutanée à circulation continue (700) selon la revendication 1, dans lequel l'endoprothèse (228) inclut une ou plusieurs feuillets d'une valve pour remplacer l'une d'une valve aortique ou d'une valve mitrale.
